(19) Europäisches Patentamt
European Patent Office
Office européen des brevets

(11) Veröffentlichungsnummer: **0 300 290 B1**

(12) **EUROPÄISCHE PATENTSCHRIFT**

(45) Veröffentlichungstag der Patentschrift: **18.12.91**

(21) Anmeldenummer: **88110926.8**

(22) Anmeldetag: **08.07.88**

(51) Int. Cl.5: **C07C 229/02**, C07C 229/22, C07C 229/28, C07C 229/34, C07C 227/00, A61K 31/19, A23K 1/22

(54) **Phenoxypropanolamine.**

(30) Priorität: **21.07.87 CH 2762/87**

(43) Veröffentlichungstag der Anmeldung:
**25.01.89 Patentblatt 89/04**

(45) Bekanntmachung des Hinweises auf die Patenterteilung:
**18.12.91 Patentblatt 91/51**

(84) Benannte Vertragsstaaten:
**CH DE FR GB IT LI**

(56) Entgegenhaltungen:
**US-A- 4 338 333**

(73) Patentinhaber: **F. HOFFMANN-LA ROCHE AG**
**Postfach 3255**
**CH-4002 Basel(CH)**

(72) Erfinder: **Alig, Leo, Dr.**
**Liebrütistrasse 32**
**CH-4303 Kaiseraugst(CH)**
Erfinder: **Müller, Marcel, Dr.**
**Quellenweg 10**
**CH-4402 Frenkendorf(CH)**

(74) Vertreter: **Mahé, Jean et al**
**Postfach 3255 Grenzacherstrasse 124**
**CH-4002 Basel(CH)**

EP 0 300 290 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Phenoxypropanolamine, Verfahren zu ihrer Herstellung und pharmazeutische Präparate auf der Basis dieser Verbindungen, sowie die Verwendung dieser Verbindungen bei der Bekämpfung bzw. Verhütung von Krankheiten.

Die obigen Phenoxypropanolamine sind die [p-[2-[[(S)-2-Hydroxy-3-phenoxypropyl]amino]äthyl]-phenoxy]essigsäure (im folgenden HPE) und die physiologisch verträglichen Salze davon. Beispiele für solche Salze sind Alkalimetall-, Erdalkalimetall-, Ammonium- und Alkylammoniumsalze, wie Na-, K-, Ca-, Trimethylammonium- und Aethanolammoniumsalze, sowie Salze der HPE mit Mineralsäuren, wie Salzsäure, Bromwasserstoffsäure, Schwefelsäure oder Phosphorsäure, oder mit organischen Säuren, wie Oxalsäure, Methansulfonsäure, Essigsäure, Propionsäure, Zitronensäure, Maleinsäure, Bernsteinsäure, Aepfelsäure, Fumarsäure, Phenylessigsäure oder Salicylsäure. Bevorzugt ist das HPE-Hydrochlorid.

Die obigen Phenoxypropanolamine können dadurch hergestellt werden, dass man einen der [p-[2-[[(S)-2-Hydroxy-3-phenoxypropyl]amino]äthyl]phenoxy]essigsäure entsprechenden Ester spaltet und gewünschtenfalls die erhaltene Säure in ein Salz überführt.

Als Ester kommen z.B. nieder-Alkylester oder der Benzylester der HPE in Frage. Die nieder-Alkylester kann man durch Verseifung unter sauren oder basischen Bedingungen abspalten, beispielsweise mit einer starken Säure, wie Salzsäure, Schwefelsäure oder Phosphorsäure, zweckmässig bei einer Temperatur zwischen 20 und 110° C, in einem polaren Lösungsmittel, wie Wasser, einem $C_{1-4}$-Alkanol, z.B. Methanol oder Aethanol; oder mit einer Base, wie Natrium- oder Kaliumhydroxyd, zweckmässig bei einer Temperatur zwischen 10 und 110°, in einem geeigneten Lösungsmittel wie einem $C_{1-4}$Alkanol. Den Benzylester der HPE kann man durch katalytische Hydrierung spalten. Zweckmässig lässt sich die HPE in Form eines Salzes aus dem Reaktionsgemisch isolieren. Die freie HPE kann man durch Extraktion aus dem wässrigen Reaktionsmedium mit einem organischen Lösungsmittel, wie Methylenchlorid oder Chloroform, bei pH 6 bis 7 isolieren.

Die Ausgangsester kann man in an sich bekannter Weise, z.B. ausgehend von (S)-Phenylglycidäther und Tyramin über das p-[2-[[(S)-2-Hydroxy-3-phenoxypropyl]amino]äthyl]phenol und Umsetzung von diesem Phenol mit einem Halogenessigsäure-niederalkylester, wie Bromessigsäuremethylester, herstellen. Letztere Umsetzung lässt sich z.B. in einem Lösungsmittel, wie Dimethylsulfoxyd, in Gegenwart einer Base, wie Kalium-t-butylat, bei einer Temperatur bis zur Rückflusstemperatur des Reaktionsgemisches bewerkstelligen. Alternativ kann man die Ausgangsester durch Reaktion des (S)-Phenylglycidäthers mit einem p-[2-Aminoäthyl]phenoxyessigsäure-niederalkylester herstellen.

Die erfindungsgemässen Verbindungen können als Wirkstoffe in pharmazeutischen Präparaten zur Behandlung der Fettsucht und/oder des Diabetes mellitus, insbesondere des obesen erwachsenen Diabetikers verwendet werden. Im Tierexperiment wurde auf Verabreichung der erfindungsgemässen Verbindungen ein gesteigerter Katabolismus, vor allem der Fette, beobachtet. Weiterhin wurde beobachtet, dass die erfindungsgemässen Verbindungen die Bildung von braunem Fettgewebe bei Ratten und obes-hyperglykämischen Mäusen stimulieren. Bekanntlich wird Defekten des braunen Fettgewebes eine wesentliche Rolle bei der Entstehung der Fettsucht zugeschrieben. An obes-hyperglykämischen Mäusen und an Streptozotocin-diabetischen Ratten haben die erfindungsgemässen Verbindungen einen ausgeprägten antidiabetischen Effekt, indem sie hypoglykämisch wirken und die Glykosurie vermindern. Die erfindungsgemässen Verbindungen zeigen nur eine geringe Wirkung auf Herztätigkeit und Kreislauf. Die Dosierung kann in Abhängigkeit von der Wirkungsstärke der einzelnen Verbindungen und den individuellen Bedürfnissen des Patienten 0,5-1000 mg, vorzugsweise 2-200 mg pro Tag für einen Erwachsenen betragen, wobei die Dosis als Einzeldosis oder in mehreren Dosen über den Tag verteilt verabreicht werden kann.

Ferner konnte mit den erfindungsgemässen Verbindungen im Tierexperiment eine Erhöhung des Proteingehaltes und eine Erniedrigung des Fettgehalts im Körper nachgewiesen werden. Die erfindungsgemässen Verbindungen führen demnach zu einer Erhöhung der mageren Körpermasse auf Kosten des Fettanteils. Daher können die erfindungsgemässen Verbindungen in der Humanmedizin zur Behandlung von Zuständen, die mit erhöhtem Proteinabbau verbunden sind, z.B. bei Rekonvaleszenz nach Operationen, verwendet werden. Dabei sind die Verabreichungsdosen die gleichen wie bei der Behandlung der Fettsucht und/oder des Diabetes mellitus.

Die erfindungsgemässen Verbindungen können auch in der Ernährung von Masttieren, wie Rindern, Schweinen, Schafen und Geflügel, Verwendung finden. Dabei können die Verabreichungsdosen und Verabreichungsformen die gleichen sein wie für Vitamine. Die erfindungsgemässen Verbindungen können auch als Futterzusatz in Dosen von 0,01-100 mg/kg je nach Substanz, Tierart und Alter eingesetzt werden.

Die pharmazeutischen Präparate enthalten den Wirkstoff zusammen mit einem verträglichen pharmazeutischen, organischen oder anorganischen Trägermaterial, wie z.B. Wasser, Gelatine, Gummi arabicum,

Lactose, Stärke, Magnesiumstearat, Talk, pflanzliche Oele, Polyalkylenglykole, Vaseline und dergleichen. Die pharmazeutischen Präparate werden vorzugsweise oral, z.B. in Form von Tabletten, Kapseln, Pillen, Pulver, Granulaten, Lösungen, Sirupen, Suspensionen, Elixiren und dergleichen verabreicht. Die Verabreichung kann aber auch parenteral, z.B. in form von sterilen Lösungen, Suspensionen oder Emulsionen, erfolgen. Die pharmazeutischen Präparate können sterilisiert sein und/oder Bestandteile enthalten, wie Konservierungsmittel, Stabilisatoren, Netzmittel, Emulgatoren, Salze, um den osmotischen Druck zu variieren, und Puffersubstanzen.

Die Aktivität der erfindungsgemässen Verbindungen wird aus den nachstehenden Versuchsresultaten deutlich:

Wirkung auf den Sauerstoffverbrauch

Männliche Albinoratten im Gewicht von 160-180 g wurden nach 24 Stunden Fasten in Stoffwechselkäfige gesetzt. Die Käfige wurden mit konstant 6 Liter Raumluft/Minute, die bei einem Taupunkt von 11 °C äquilibriert wurde, belüftet. Von der Abluft wurde nach erneuter Aequilibrierung während Perioden von jeweils 14 Minuten Proben gesammelt und der S Sauerstoff- und $CO_2$-Gehalt analysiert. Nach einer Anpassungszeit von 4 Stunden erhielten die in Gruppen zu 6 aufgeteilten Tiere entweder Placebo (5% Gummi arabicum) oder die Testsubstanz, nämlich das HPE-Hydrochlorid (suspendiert in 5% Gummi arabicum) per os. Danach wurden 12 Stunden lang die Bestimmungen durchgeführt. In der nachfolgenden Tabelle ist der Prozentsatz des gemittelten Sauerstoffverbrauchs nach Medikation während der ersten 3 Stunden und der gesamten Versuchsdauer (12 Stunden) vom Sauerstoffverbrauch der Anpassungsperiode angegeben, wobei entsprechende Korrekturen für Aenderungen in der Placebo-Gruppe berücksichtigt wurden.

Tabelle

| Dosis (µM/kg) | Sauerstoffverbrauch (% vom Wert der Vorperiode) | |
|---|---|---|
| | 1. bis 3. Stunde | 1. bis 12. Stunde |
| 0,03 | 114 | 105 |
| 0,1 | 132 | 105 |
| 0,3 | 152 | 117 |
| 1 | 169 | 129 |

Erst bei Verabreichung von etwa 100 µM/kg tritt eine geringe chronotrope Wirkung (Tachycardie) ein.

Die folgenden Beispiele erläutern die Erfindung weiter.

Beispiel 1

1,90 g [p-[2-[[(S)-2-Hydroxy-3-phenoxypropyl]amino] äthyl]phenoxy]essigsäuremethylester wurden mit 80 ml 5 % methanol. KOH und 80 ml Wasser 90 Min. auf 50° erwärmt. Die Reaktionslösung wurde abgekühlt auf Eiswasser gegossen und mit Aether extrahiert. Die wässerig-alkalische Phase wurde mit konz. HCl auf pH 2 angesäuert und im Vakuum bie 40° C zur Trockene eingedampft. Der Rückstand wurde mit Toluol im Vakuum eingedampft. Der Rückstand wurde mit 150 ml eines Gemisches von Aethanol-Methanol 2:1 verrührt, der Niederschlag wurde abfiltriert und das Filtrat im Vakuum eingedampft. Der Rückstand wurde in 20 ml Methanol gelöst und mit 20 ml Aether versetzt. Es kristallisierten 1,5 g [p-(2-[[(S)-2-Hydroxy-3-phenoxypropyl]amino]äthyl]phenoxy]essigsäure-hydrochlorid, Schmelzpunkt 194 - 196° C (Zers.),

$$[\alpha]_D^{20°} = -18°$$

(c = 0,5 in Methanol).

Das Ausgangsmaterial wurde wie folgt hergestellt:

Reaktion von S-Phenylglycidäther mit Tyramin ergab p-[2-[[(S)-2-Hydroxy-3-phenoxypropyl]amino]äthyl]-phenol vom Smp 118 - 119° C. Dieses wurde mit Bromessigsäuremethylester und Kalium-t-butylat in

DMSO bei Raumtemperatur zu amorphem [p-[2-[[(S)-2-Hydroxy-3-phenoxypropyl]amino]äthyl]phenoxy]-essigsäuremethylester umgesetzt. Dieses wurde als Hydrochlorid vom Smp. 167 - 168° C aus Aceton kristallisiert,

$$[\alpha]_D^{20^\circ} = -16^\circ$$

(C = 1,0 in Methanol).

Beispiel 2

382 mg HPE-Hydrochlorid wurden in 16 ml Aethanol und 4 ml Methanol gelöst und mit 80 mg Natriumhydroxid versetzt. Die Lösung wurde 1 Stunde im Kühlschrank gehalten, wobei man 270 mg HPE-Natriumsalz, Schmelzpunkt 200-201°,

$$[\alpha]_D^{20^\circ} = -4,5^\circ$$

(c = 0,5 in Methanol) erhielt.

Beispiel 3

382 mg HPE-Hydrochlorid wurden in 5 ml heissem Methanol gelöst, mit 40 mg Natriumhydroxid versetzt und abgekühlt. Das ausgefallene NaCl wurde abfiltriert und das Filtrat mit 130 mg Maleinsäure versetzt. Dann gab man 15 ml Aether dazu und liess im Kühlschrank kristallisieren. Man erhielt 300 mg HPE-Maleinat vom Schmelzpunk 163-166°,

$$[\alpha]_D^{20^\circ} = -15^\circ$$

(c = 0,6 in Methanol).

Beispiel 4

Es werden in üblicher Weise Tabletten folgender Zusammensetzung hergestellt:

| | |
|---|---|
| [p-[2[[(S)-2-Hydroxy-3-phenoxypropyl]amino]äthyl]phenoxy]essigsäure-hydrochlorid. | 250 mg |
| Lactose | 200 mg |
| Maisstärke | 300 mg |
| Maisstärkepaste | 50 mg |
| Calciumstearat | 5 mg |
| Dicalciumphosphat | 45 mg |

## Patentansprüche

1. [p-[2[[(S)-2-Hydroxy-3-phenoxypropyl]amino]äthyl]phenoxy]essigsäure und die physiologisch verträglichen Salze davon.

2. [p-[2[[(S)-2-Hydroxy-3-phenoxypropyl]amino]äthyl]phenoxy]essigsäure-hydrochlorid.

3. Verbindungen nach Anspruch 1 oder 2 als therapeutische Wirkstoffe, insbesondere zur Behandlung der Fettsucht, des Diabetes mellitus und von Zuständen, die mit erhöhtem Proteinabbau verbunden sind.

4. Pharmazeutische Präparate, gekennzeichnet durch einen Gehalt an einer Verbindung nach Anspruch 1 oder 2.

5. Futtermittel für Masttiere enthaltend eine Verbindung nach Anspruch 1 oder 2.

6. Verfahren zur Herstellung der Verbindungen nach Anspruch 1 oder 2, dadurch gekennzeichnet, dass man einen der [p-[2[[(S)-2-Hydroxy-3-phenoxypropyl]amino]äthyl]phenoxy]essigsäure entsprechenden Ester spaltet und gewünschtenfalls die erhaltene Säure in ein Salz überführt.

7. Verwendung einer Verbindung nach Anspruch 1 oder 2 zur Herstellung von pharmazeutischen Präparaten zur Behandlung der Fettsucht, des Diabetes mellitus und von Zuständen, die mit erhöhtem Proteinabbau verbunden sind.

## Claims

1. [p-[2-[[(S)-2-Hydroxy-3-phenoxypropyl]amino]ethyl]-phenoxy]acetic acid and the physiologically compatible salts thereof.

2. [p-[2-[[(S)-2-Hydroxy-3-phenoxypropyl]amino]ethyl]-phenoxy]acetic acid hydrochloride.

3. Compounds according to claim 1 or 2 as therapeutically active substances, especially for the treatment of obesity, of diabetes mellitus and of conditions which are associated with an increased protein breakdown.

4. Pharmaceutical preparations containing a compound according to claim 1 or 2.

5. Feedstuffs for fattening animals containing a compound according to claim 1 or 2.

6. A process for the manufacture of the compounds according to claim 1 or 2, characterized by cleaving an ester corresponding to [p-[2-[[(S)-2-hydroxy-3-phenoxypropyl]amino]-ethyl]phenoxy]acetic acid and, if desired, converting the acid obtained into a salt.

7. The use of a compound according to claim 1 or 2, for the manufacture of pharmaceutical preparations for the treatment of obesity, of diabetes mellitus and of conditions which are associated with an increased protein breakdown.

## Revendications

1. L'acide [p-[2-[[(S)-2-hydroxy-3-phénoxypropyl]-amino]-éthyl]-phénoxy]- acétique et ses sels acceptables pour l'usage pharmaceutique.

2. Le chlorhydrate de l'acide [P-[2-[[(s)-2-hydroxy-3-phénoxypropyl]-amino]-éthyl]-phénoxy]-acétique.

3. Les composés de la revendication 1 ou 2, en tant que substances actives thérapeutiques, en particulier pour le traitement de l'obésité, du diabète sucré et des états associés à une dégradation accrue des protéines.

4. Compositions pharmaceutiques caractérisées en ce qu'elles contiennent un composé selon la revendication 1 ou 2.

5. Aliments pour animaux à l'engrais, contenant un composé selon la revendication 1 ou 2.

6. Procédé de préparation des composés de la revendication 1 ou 2, caractérisé en ce que l'on scinde un ester correspondant à l'acide [p-[2-[[(S)-2-hydroxy-3-phénoxypropyl]-amino]-éthyl]-phénoxy]-acétique et, si on le désire, on convertit l'acide obtenu en un sel.

7. Utilisation d'un composé selon la revendication 1 ou 2 pour la préparation de compositions pharmaceutiques pour le traitement de l'obésité, du diabète sucré et des états associés à une dégradation accrue des protéines.